# EUROPEAN PATENT APPLICATION

(11) **EP 3 628 270 A1**
(43) Date of publication of application: **01.04.2020**
(21) Application number: 19199728.7
(22) Date of filing: 26.09.2019
(51) Int. Cl.: A61C 8/00, A61C 1/18, A61C 19/04, A61B 17/68

(54) **DENTAL TORQUE WRENCH**

(30) Priority: 27.09.2018 JP 2018181188
(71) Applicant: GC Corporation, Sunto-gun, Shizuoka 410-1307 (JP)
(72) Inventor: HONMA, Tatsuro, Itabashi-ku,, Tokyo (JP); SUGINO, Misato, Itabashi-ku, Tokyo (JP)
(74) Representative: Müller-Boré & Partner Patentanwälte PartG mbB

(57) **Abstract**

A dental torque wrench (1) includes a main body (10) having a head (11), and a body (12); a main shaft (30) held inside the body (12); a pin (33) having a pin body (331), the pin (33) extending along an axial direction of the main shaft (30); a clamp member (13) having a predetermined opening in an axial view of the pin (33), the clamp member (13) being disposed to be sandwiched between an inner circumferential surface of the body (12) and an outer circumferential surface of an insertion portion (1122) of the head (11), the insertion portion (1122) being inserted into an internal space of the body (12); at least one projected portion (333) on a side surface of the pin body (331); and a first slit portion (1161) formed on a side surface of the insertion portion (1122) to receive the projected portion (333).

## Description

### BACKGROUND OF THE INVENTION

### 1. Field of the Invention

The disclosures discussed herein relate to a dental torque wrench.

### 2. Description of the Related Art

In typical implant treatment, a support is fixed by a screw to a tooth root implanted in the jawbone, and an artificial tooth is attached to this fixed support. To fix the support to the implanted tooth root by tightening a screw, the application of a small torque may cause the attached artificial tooth to rattle, but the application of a large torque may damage the jawbone. In order to avoid such drawbacks, a dental torque wrench is used to attach a support to the implanted tooth root. The dental torque wrench is enabled to tighten a screw at a predetermined torque.

Such a dental torque wrench includes a head and a body. To tighten a screw by using a typical dental torque wrench, a threaded tool is attached to a hole of the head. In the hole of the head, a pawl is protruded. By rotation of the dental torque wrench in one rotational direction, the protruded pawl engages with the threaded tool to rotate the threaded tool. By rotation of the dental torque wrench in an opposite rotational direction, the pawl does not engage with the threaded tool, allowing the dental torque wrench to spin out. That is, the typical dental torque wrench has a so-called ratchet mechanism (feeding function) operated by a pawl.

The dental torque wrench with a ratchet mechanism is thus enabled to switch between tightening a screw and loosening a screw by reversing rotational directions of the dental torque wrench by 180 degrees. For example, according to the torque wrench disclosed in Patent Document 1, a bolt rod inserted into a hole of a ratchet handle is drawn from the ratchet handle and the bolt rod is rotated by 180 degrees. The bolt rod is then put back into the ratchet handle to engage a catch with the ratchet head of a ratchet tool via the bolt rod, thereby reversing the rotational direction of the torque wrench.

However, the torque wrench disclosed in Patent Document 1 appears to fail to include a configuration in which the bolt rod is maintained while the bolt rod being inserted in a hole of the ratchet handle and a configuration in which amount of force required for inserting and drawing the bolt rod is reduced.

### [Related-Art Document]

### [Patent Document]

[Patent Document 1] Japanese Patent No. 2966772

### SUMMARY OF THE INVENTION

Accordingly, an aspect of the present invention is intended to provide a dental torque wrench configured to stably maintain attachment between a head and a body upon attaching the head and the body, and to easily detach the head and the body upon detaching the head and the body.

According to one embodiment of the present invention, a dental torque wrench (1) includes
a main body (10) having a head (11), and a body (12), the head (11) being coupled to a tool (60), and the body (12) being detachably coupled to the head (11) ;
a main shaft (30) configured to be held in a state of being inserted into the body (12);
a pin (33) having a pin body (331), the pin (33) being disposed at one end portion of the main shaft (30), the one end portion of the main shaft (30) being located close to the head (11), and the pin (33) extending along an axial direction of the main shaft (30) ;
a clamp member (13) having a predetermined opening in an axial view of the pin (33), the clamp member (13) being disposed to be sandwiched between an inner circumferential surface of the body (12) and an outer circumferential surface of an insertion portion (1122) of the head (11), the insertion portion (1122) being inserted into an internal space of the body (12);
at least one projected portion (333) disposed on a side surface of the pin body (331), the projected portion (333) being projected in a radial direction of the pin body (331); and
a first slit portion (1161) formed on a side surface of the insertion portion (1122), the first slit portion (1161) being configured to allow the projected portion (333) to be detachably inserted in the first slit portion (1161), wherein
upon coupling of the head (11) and the body (12), the pin (33) is inserted into the insertion portion (1122) of the head (11) without allowing the projected portion (333) to engage with the first slit portion (1161), the clamp member (13) is disposed to be sandwiched between the inner circumferential surface of the body (12) and the outer circumferential surface of the insertion portion (1122), and the projected portion (333) subsequently engages with the first slit portion (1161) to lock the head (11) and the body (12).

### [Advantageous Effects of Invention]

According to an aspect of the following embodiments, a dental torque wrench is configured to maintain a stable attachment of a head and a body upon attaching the head and body, and to easily detach the head from the body upon detaching the head and the body.

### BRIEF DESCRIPTION OF THE DRAWINGS

Other objects and further features of the present invention will be apparent from the following detailed description when read in conjunction with the accompanying drawings, in which:
FIG. 1 is a perspective view illustrating a dental torque wrench according to an embodiment;
FIG. 2 is a cross-sectional view taken along I-I of FIG. 1;
FIG. 3 is a perspective view illustrating a state in which a head and a body of a dental torque wrench main body are separated;
FIG. 4 is a partially enlarged view from a II-II direction of FIG. 3;
FIG. 5 is a cross-sectional view from a III-III direction of FIG. 2;
FIG. 6 is a cross-sectional view from a IV-IV direction of FIG. 2;
FIG. 7 is a cross-sectional view from a V-V direction of FIG. 1;
FIG. 8 is a front view illustrating an example of a tool that is locked to a dental torque wrench;
FIG. 9 is a diagram illustrating a step in a attaching procedure between a head and a body in a dental torque wrench main body;
FIG. 10 is a diagram illustrating a step in the attaching procedure between the head and the body in the dental torque wrench main body;
FIG. 11 is a diagram illustrating a step in a detaching procedure between a head and a body of a dental torque wrench main body;
FIG. 12 is a diagram illustrating a step in the detaching procedure between the head and the body of the dental torque wrench main body;
FIG. 13 is a partially enlarged sectional view illustrating a state in which a tool is locked to a head of a dental torque wrench illustrated in FIG. 2;
FIG. 14 is a diagram illustrating a step in an inversion procedure of a ratchet mechanism in a dental torque wrench;
FIG. 15 is a diagram illustrating a step in the inversion procedure of the ratchet mechanism in the dental torque wrench; and
FIG. 16 is a diagram illustrating a step in the inversion procedure of the ratchet mechanism in the dental torque wrench.

### EMBODIMENT FOR CARRYING OUT THE INVENTION

Hereinafter, embodiments of the present invention will be described in detail with reference to the accompanying drawings. In order to facilitate the understanding of the following description, duplicate descriptions are omitted with reference to the same reference numerals for the same components in the drawings. The scale of each member in the figures may differ from the actual scale. In the following description, a three-dimensional orthogonal coordinate system in three axial directions (X-axis, Y-axis, and Z-axis directions) is used for illustrating embodiments. In the three axial directions, an X-axis direction indicates a direction parallel to an axial direction of a body of a dental torque wrench main body. Of the X-axis direction, the direction toward a head of the dental torque wrench main body is defined as a positive direction (+X-axis direction), and the direction toward a knob of the dental torque wrench main body is defined as a negative direction (-X-axis direction). With respect to a plane perpendicular to the axial direction of the body of the dental torque wrench, one of two directions perpendicular to each other is defined as a Y-axis direction, and the other direction is defined as a Z-axis direction. The Y-axis direction indicates an axial direction of a locking portion of the head, and the Z-axis direction indicates an extending direction of a graduation portion.

### <Dental Torque Wrench>

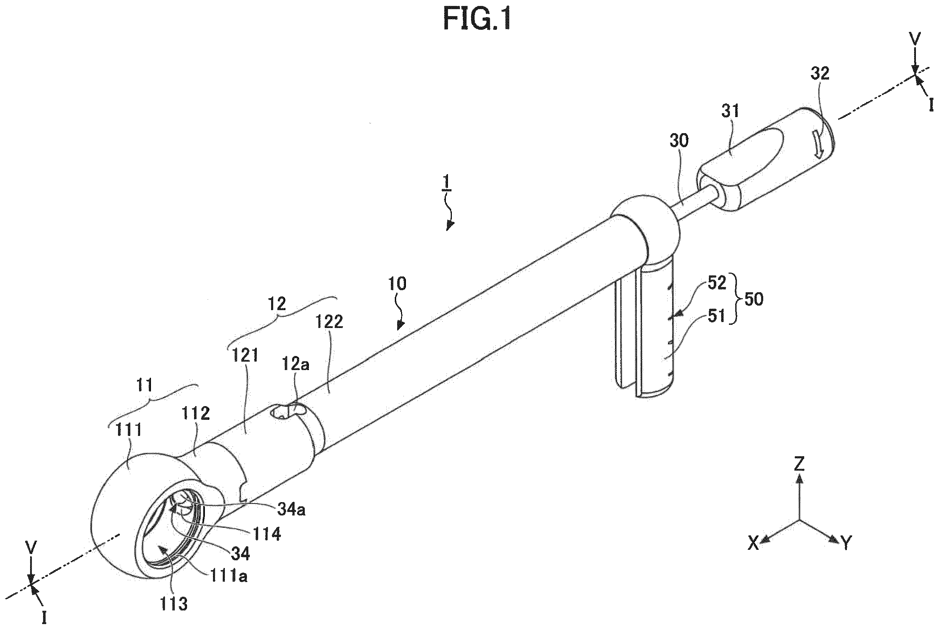

The following illustrates a configuration of a dental torque wrench according to an embodiment. FIG. 1 is a perspective view illustrating a dental torque wrench according to an embodiment. As illustrated in FIG. 1, a dental torque wrench 1 includes a main body 10, a main shaft 30, and a graduation portion 50. Hereinafter, respective members constituting the dental torque wrench 1 will be described.

### [Main Body]

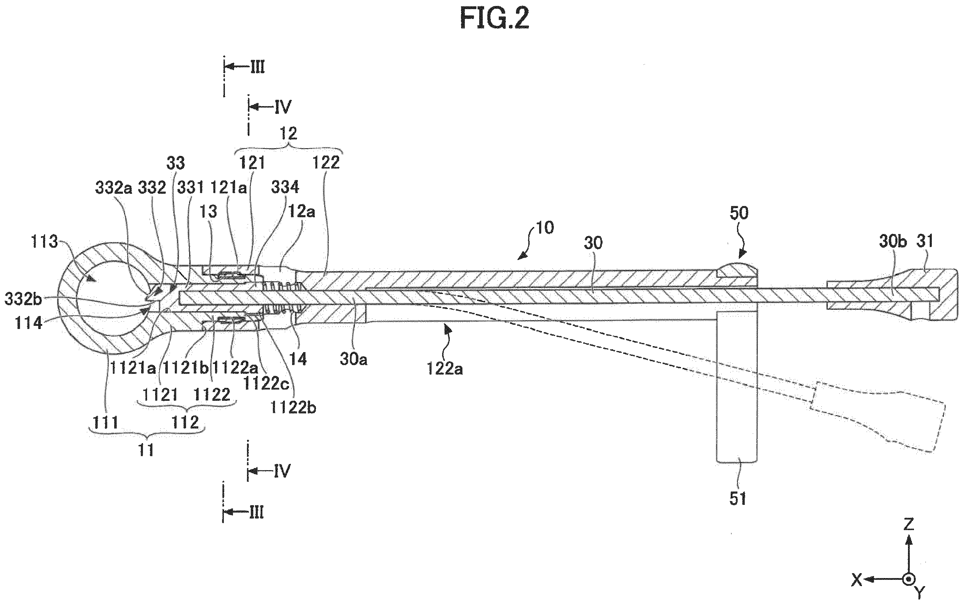

FIG. 2 is a cross-sectional view taken along I-I of FIG. 1. As illustrated in FIG. 2, the main body 10 of the dental torque wrench 1 includes a head 11, a body 12, a C-ring 13 as a clamp member, and a coil spring 14.

### (Head)

As illustrated in FIG. 2, the head 11 includes a cylindrical locking portion 111 axially oriented in the Y-axis direction, and a cylindrical connection portion 112 coupled to a side surface of the locking portion 111. The axial direction of the cylindrical connection portion 112 is in the X-axis direction.

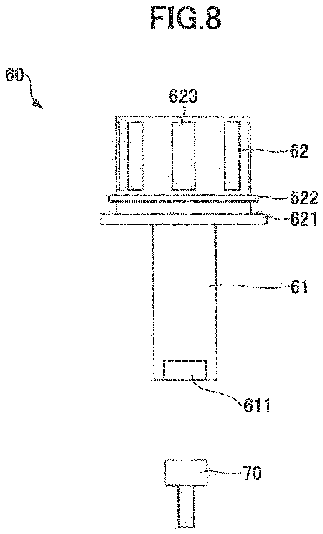

As illustrated in FIG. 2, the locking portion 111 has a housing portion 113, which is a space surrounded by an inner circumferential surface of the locking portion 111. A tool 60 (see FIG. 8) is coupled to the locking portion 111. The locking portion 111 has a substantially circular opening 114 on its inner circumferential surface to connect the housing portion 113 and an internal space of the connection portion 112.

As illustrated in FIG. 2, the connection portion 112 extends from the locking portion 111 toward a radial direction of the locking portion 111. The connection portion 112 includes a contact portion 1121 connected to the locking portion 111, and an insertion portion 1122 connected to the contact portion 1121, in this order from the locking portion 111. The insertion portion 1122 has an outer diameter smaller than an outer diameter of the contact portion 1121.

As illustrated in FIG. 2, the contact portion 1121 is formed in a cylindrical shape in an axial view. The contact portion 1121 has a hole 1121a that is connected to the opening 114 of the locking portion 111. The size of the hole 1121a is determined based on an inner diameter of the contact portion 1121. The inner diameter of the contact portion 1121 is substantially equal to or greater than an outer diameter of a pin body 331 of a pin 33.

The contact portion 1121 has an end face 1121b next to the insertion portion 1122 of the connection portion 112. The end face 1121b is formed in a plane substantially perpendicular to an extending direction (X-axis direction) of the connection portion 112. The end face 1121b is an interface portion between the contact portion 1121 and insertion portion 1122.

As illustrated in FIG. 2, the insertion portion 1122 is formed in a cylindrical shape. The insertion portion 1122 has a groove portion 1122a formed on its outer circumferential surface along a circumferential direction. The groove portion 1122a receives a C-ring 13. When the insertion portion 1122 is inserted into the fitting portion 121, the groove portion 1122a is located to face a groove portion 121a formed an inner surface of a fitting portion 121 along a circumferential direction. The C-ring 13 is disposed in a space formed by these groove portions 1122a and 121a, as will be described below.

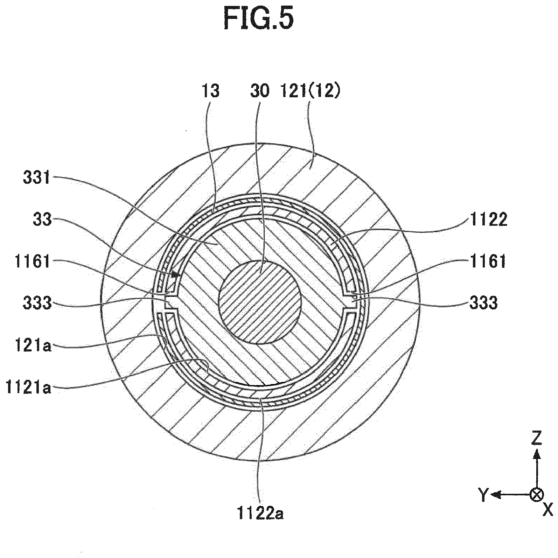

In a portion of the insertion portion 1122 where the groove portion 1122a is formed, the inner diameter of the insertion portion 1122 is substantially equal to or greater than the outer diameter of the pin body 331 of the pin 33, but is less than the sum of the outer diameter of the pin body 331 and the heights of the two projected portions 333 (see FIG. 5).

The insertion portion 1122 has two end portions, one end portion located close to the locking portion 111 and the other end portion located opposite to the locking portion 111 (-X-axis direction). The insertion portion 1122 includes an enlarged diameter portion 1122b at the other end portion into which the pin 33 is inserted. The enlarged diameter portion 1122b has an inner diameter larger than an inner diameter at the groove portion 1122a of the insertion portion 1122. The enlarged diameter portion 1122b of the insertion portion 1122 has an inner diameter larger than inner diameters of other portions of the insertion portion 1122. The inner diameter of the enlarged diameter portion 1122b is also larger than an enlarged portion 334 of the pin 33. In the above configuration, the pin 33 is rotatable in its axial direction in the enlarged diameter portion 1122b of the insertion portion 1122. Note that the inner diameter of the enlarged diameter portion 1122b may be substantially the same as the sum of the outer diameter of the pin body 331 of the pin 33 and heights of the two projected portions 333; or the inner diameter of the enlarged diameter portion 1122b may be substantially the same as the outer diameter of the enlarged portion 334 of the pin 33.

The insertion portion 1122 has a step 1122c at which an inner diameter increases between the groove portion 1122a and the other end portion located opposite to the locking portion 111 (-X-axis direction). That is, the insertion portion 1122 includes the step 1122c between an inner circumferential surface of the groove portion 1122a and the inner circumferential surface of the enlarged diameter portion 1122b.

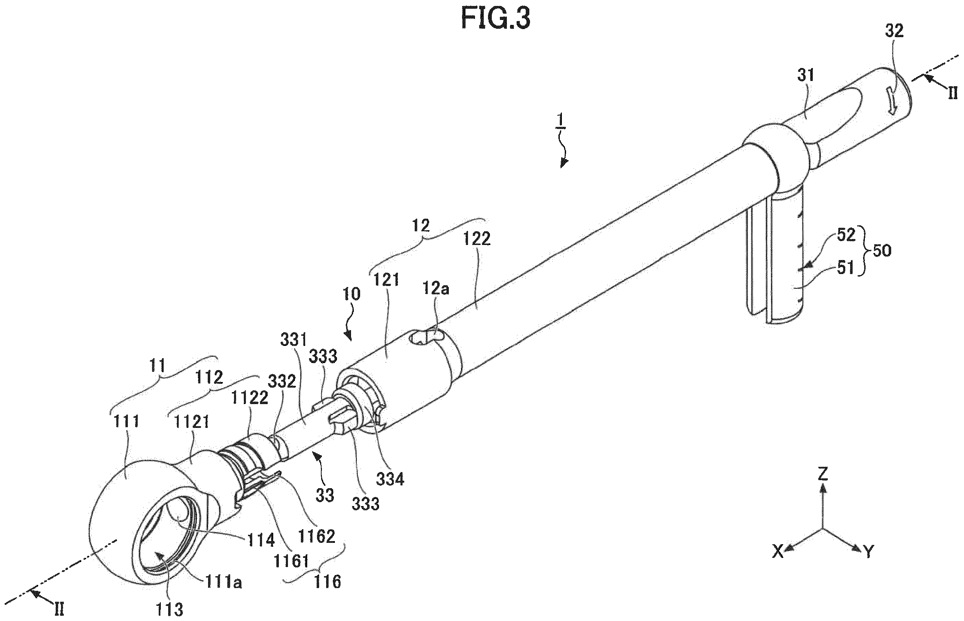

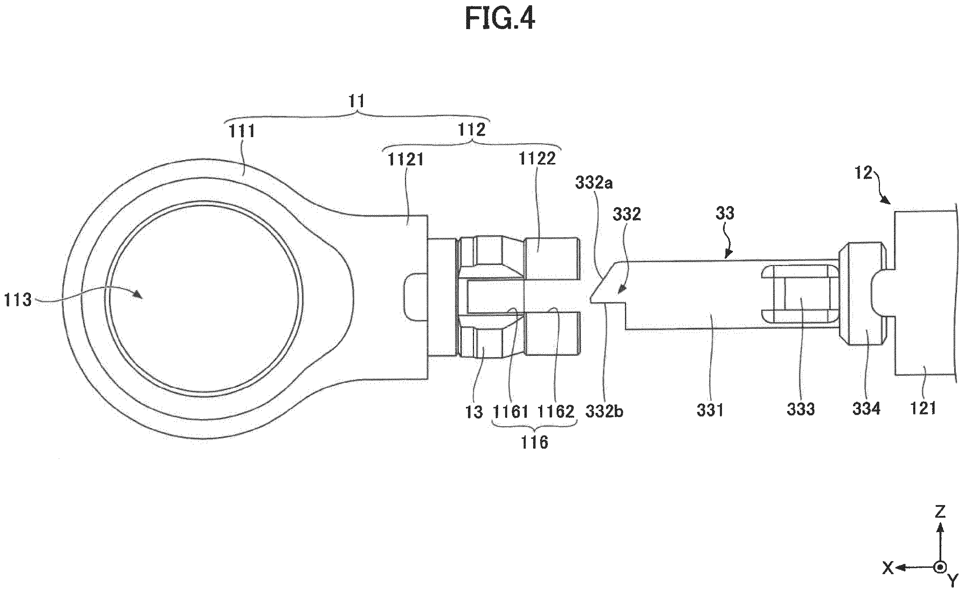

FIG. 3 is a perspective view illustrating a state in which the head 11 is detached from the body 12 in the main body 10 of the dental torque wrench 1 illustrated in FIG. 1, and FIG. 4 is a partially enlarged view illustrating a state in which the head 11 and the body 12 are viewed from the II-II direction of FIG. 3. As illustrated in FIGS. 3 and 4, the insertion portion 1122 has slit portions 116 on its side surface. A pair of the slit portions 116 is formed on a side surface of the insertion portion 1122 such that the slit portions 116 are disposed opposite to each other.

The slit portions 116 are formed on part of the side surface of the insertion portion 1122 along an axial direction from the other end portion of the insertion portion 1122 (-X-axis direction side) toward the locking portion 111.

The slit portions 116 include first slit portions 1161 formed in the groove portion 1122a, and second slit portions 1162 formed between the groove portion 1122a and the other end portion opposite to the locking portion 111.

FIG. 5 is a cross-sectional view when viewed from the III-III direction of FIG. 2. As illustrated in FIG. 5, the inner diameter of the insertion portion 1122 having the first slit portions 1161 is smaller than the sum of the outer diameter of the pin body 331 of the pin 33 and the heights of the two projected portions 333. The width of each first slit portion 1161 is larger than the width of the corresponding projected portion 333. Thus, the first slit portions 1161 are formed so as to allow the projected portions 333 of the pin 33 to be inserted into or removed from the first slit portions 1161, respectively. Note that the "width" in this case refers to a circumferential length of a first slit portion 1161 or a projected portion 333.

The second slit portions 1162 illustrated in FIGS. 3 and 4 are formed from the groove portion 1122a to the other end portion opposite to the locking portion 111; that is, the second slit portions 1162 are formed in the part of the insertion portion 1122 forming the enlarged diameter portion 1122b, from the groove portion 1122a. The second slit portions 1162 are connected to the first slit portions 1161 as illustrated in FIGS. 3 and 4.

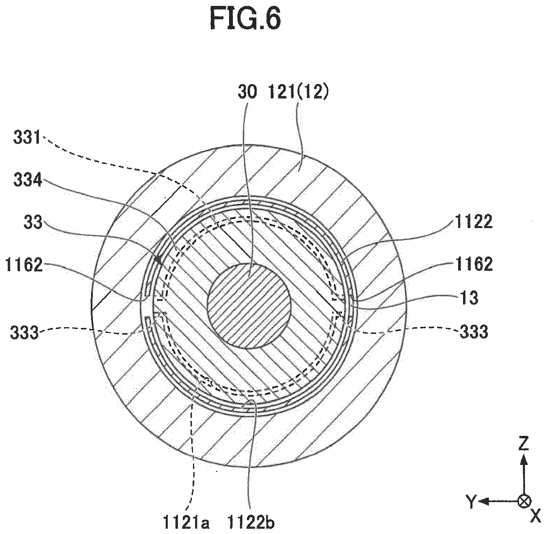

FIG. 6 is a cross-sectional view when viewed from the IV-IV direction of FIG. 2. As illustrated in FIG. 6, the inner diameter of the enlarged diameter portion 1122b is equal to or larger than the sum of the outer diameter of the pin body 331 of the pin 33 and heights of the two projected portions 333, and is equal to or larger than the outer diameter of the enlarged portion 334 of the pin 33. Hence, upon inserting the pin 33 into the insertion portion 1122, the pin 33 does not contact the second slit portions 1162.

The width of the second slit portion 1162 is, as illustrated in FIG. 4, larger than the width of the projected portion 333 as in the first slit portion 1161.

### (Body)

As illustrated in FIGS. 1 and 2, the body 12 is a cylindrical member and includes a fitting portion 121 and a small diameter portion 122. The fitting portion 121 is detachably coupled to the connection portion 112 of the head 11.

As illustrated in FIGS. 1 and 2, the fitting portion 121 has an outer diameter larger than an outer diameter of the small diameter portion 122 from one end to a predetermined position along an extending direction. The insertion portion 1122 of the connection portion 112 is inserted into the fitting portion 121.

The fitting portion 121 has an end face next to the connection portion 112, and this end face is formed in a plane substantially perpendicular to an extending direction (X-axis direction) of the body 12, as with the end face 1121b of the contact portion 1121.

The fitting portion 121 has a groove portion 121a on its inner circumferential surface along a circumferential direction. Upon the insertion portion 1122 being inserted into the fitting portion 121, the groove portion 121a faces the groove portion 1122a formed on an outer circumferential surface of the insertion portion 1122. The insertion portion 1122 and the fitting portion 121 are connected by a C-ring 13, which is disposed in a space formed between the groove portion 1122a and the groove portion 121a.

As illustrated in FIGS. 1 and 2, a small diameter portion 122 is disposed from one end portion, which is fixed to the connection portion 112 of the head 11 by a predetermined interval, to the other end portion (-X axis direction). The small diameter portion 122 has the smallest inner diameter of all the portions of the body 12. The inner diameter of the small diameter portion 122 is substantially the same as a diameter of the main shaft 30.

As illustrated in FIG. 2, the small diameter portion 122 has one end portion located close to the head 11 and the other end portion located opposite to the head 11, and the small diameter portion 122 includes a cutout 122a formed on a part of its side surface along an axial direction from the other end portion toward the head 11 (+X-axis direction). The cutout 122a is formed in a direction (-Z-axis direction) perpendicular to an extending direction of the body 12, in view from a cylindrical axis direction (Y-axis direction) of the locking portion 111. The width of the cutout 122a is larger than the diameter of the main shaft 30.

The cutout 122a may be formed from the end opposite to the head 11 to a predetermined position of the small diameter portion 122, and is not necessarily formed throughout an entire axial length of the small diameter portion 122.

### (C-ring)

As illustrated in FIG. 5, the C-ring 13 is a clamp member formed in a C shape in an axial view of the pin 33. The C-ring 13 has a ring shape and has an opening (cut) at one place in a circumferential direction of the ring. The opening angle of a slit of the C-ring 13 may be large enough to allow the C-ring 13 to deform when the C-ring 13 is pressed.

As illustrated in FIG. 2, the C-ring 13 is disposed in a groove portion 1122a of the insertion portion 1122, which is inserted into an internal space of the body 12. Upon the outer circumferential surface of the insertion portion 1122 being connected to the fitting portion 121 of the body 12, the C-ring 13 is disposed in a space formed between the groove portion 1122a and the groove portion 121a while the C-ring 13 is being sandwiched between the outer circumferential surface of the insertion portion 1122 and an inner circumferential surface of the fitting portion 121 of the body 12. The insertion portion 1122 and the fitting portion 121 are thus coupled by the C-ring 13.

The C-ring 13 has a predetermined opening in an axial view of the pin 33, as illustrated in FIG. 5. As illustrated in FIG. 2, upon the insertion portion 1122 being inserted into the fitting portion 121 while the C-ring 13 is being fitted in the groove portion 1122a, the C-ring 13 is pressed by the inner circumferential surface of the fitting portion 121. As a result, the C-ring 13 elastically deforms to close an opening of the C-ring 13, thereby reducing an outer diameter of the C-ring 13. Upon the insertion portion 1122 being inserted to a position at which the groove portion 1122a and the groove portion 121a face each other, the C-ring 13 elastically restores to be pressed against an inner circumferential surface of the body 12, thereby fixing the connection portion 112 to the body 12.

### (Coil spring)

As illustrated in FIG. 2, a coil spring 14 is an elastic body made of helically wound wire material. The coil spring 14 is retractably disposed in a space between the small diameter portion 122 of the body 12 and the pin 33 of the main shaft 30 within the internal space of the body 12. The main shaft 30 is inserted through the coil spring 14, and one end of the coil spring 14 contacts the small diameter portion 122 and the other end of the coil spring 14 contacts the pin 33. Note that the spring constant and the size of the coil spring 14 may appropriately be determined.

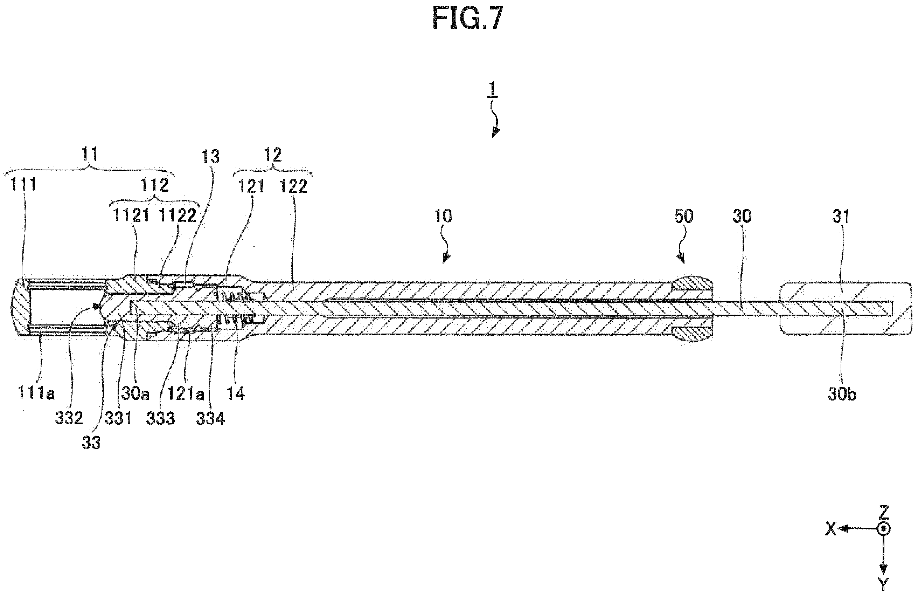

As illustrated in FIG. 2, by an elastic force of the coil spring 14, a preloading force is applied to the main shaft 30 held in the body 12 toward the head 11. FIG. 7 is a cross-sectional view from a V-V direction of FIG. 1. As illustrated in FIG. 7, when the dental torque wrench 1 is arranged as illustrated in FIG. 2, +X-axis ends of the projected portions 333 of the pin 33 contact +X-axis ends of the first slit portions 1161 (see FIGS. 3 and 4).

The space inside the body 12 in which the coil spring 14 is disposed is, as illustrated in FIG. 2, formed so as to allow the pin 33 to move in the X-axis direction from a first position to a second position, where the first position is a position at which a tip of the pawl 332 protrudes from the opening 114 toward the housing portion 113, and the second position is a position at which the enlarged portion 334 is drawn from the enlarged diameter portion 1122b.

When the main shaft 30 is moved to a position at which the projected portions 333 of the pin 33 reach a position of the enlarged diameter portion 1122b, the main shaft 30 becomes rotatable in the axial direction.

As illustrated in FIG. 2, the body 12 has window portions 12a at a position where the coil spring 14 is disposed, and the window portions 12a are configured to penetrate the internal space of the body 12. The window portions 12a are, for example, configured to penetrate the body 12 in the Z-axis direction. Thus, a pair of window portions 12a is disposed on the outer circumferential surface of the body 12 such that the window portions 12a of the pair face each other. By disposing the window portions 12a on the body 12, it is possible to visually check the position of the pin 33 inside the main body 10, expansion and contraction of the coil spring 14, and the like. Further, when the main body 10 is split into the head 11 and the body 12, cleanability and aeration of the internal space of the main body 10 may be improved.

### [Main shaft]

As illustrated in FIGS. 1 and 2, the main shaft 30 is a cylindrical member, which has elasticity against torque within a predetermined range in the radial direction of the main shaft 30. The main shaft 30 is inserted into the body 12 such that the axis of the main shaft 30 is substantially in line with a cylindrical axis of the body 12. The main shaft 30 is held inside the body 12 so as to allow the main shaft 30 to move in its axial direction and to rotate around the axis of the main shaft 30.

The diameter of the main shaft 30 is substantially the same as the inner diameter of the small diameter portion 122 of the body 12. The main shaft 30 is inserted into the small diameter portion 122 and is supported by the small diameter portion 122. The diameter of the main shaft 30 is smaller than the width of the cutout 122a of the body 12; that is, the diameter of the main shaft 30 is smaller than the width of the cutout 122a in a direction substantially perpendicular to an extending direction of the body 12 (Y-axis direction).

As illustrated in FIG. 2, one end portion 30a of the main shaft 30, which is located close to the head 11 (+X-axis side), is located inside the connection portion 112.

As illustrated in FIG. 2, the other end portion 30b of the main shaft 30 located opposite to the head 11 (-X-axis side) extends outwardly from an end of the body 12 opposite to the head 11. The other end portion 30b is provided with a knob 31.

An arrow 32 is formed on a side surface of the knob 31 as illustrated in FIG. 1. The arrow 32 indicates an orientation of an engaging surface 332b of the pawl 332 of a pin 33, which will be described later. In FIG. 1, the arrow 32 is directed to an orientation of the cutout 122a. The arrow 32 may be formed by printing or engraving.

### (Pin)

As illustrated in FIG. 2, the pin 33 is disposed on one end portion 30a of the main shaft 30 and extends along an axial direction of the main shaft 30. The pin 33 is inserted into a hole of the connection portion 112 (hole 1121a of the contact portion 1121 and a hole in the insertion portion 1122).

As illustrated in FIGS. 3 and 4, the pin 33 has a pin body 331, a pawl 332, projected portions 333, and an enlarged portion 334.

As illustrated in FIG. 2, the pin body 331 is disposed on a tip of main shaft 30. As illustrated in FIG. 3, the pin body 331 extends in the axial direction of the main shaft 30 and forms a substantially cylindrical shape.

As illustrated in FIG. 2, the pawl 332 is disposed on a tip (+X-axis side) of the pin body 331. The pawl 332 is exposed inside the housing portion 113 of the locking portion 111 so as to propagate a rotational force to a screw 70 (see FIG. 8) in one direction. The pawl 332 has an inclined surface 332a with respect to the axial direction of the main shaft 30, and an engaging surface 332b substantially parallel to the axial direction of the main shaft 30 so that the pawl 332 tapers in the cylindrical axial (Y-axis) view of the locking portion 111.

As illustrated in FIG. 2, the inclined surface 332a is located on an opposite side of the cutout 122a (the +Z-axis direction), in the cylindrical axial view of the locking portion 111.

As illustrated in FIG. 2, the engaging surface 332b is located on the same side (-Z-axis direction) as the cutout 122a, in the cylindrical axial view of the locking portion 111.

As illustrated in FIG. 2, the tip of the pawl 332 protrudes inside the housing portion 113 via an opening 114 formed on an inner circumferential surface of the locking portion 111.

An orientation of the engaging surface 332b of the pawl 332 may be specified in accordance with an orientation of the arrow 32. In FIGS. 1 and 2, the engaging surface 332b is oriented toward the cutout 122a (-Z-axis direction).

As illustrated in FIGS. 3 and 4, the pair of projected portions 333 is disposed on a side surface of the pin body 331, and the pair of projected portions 333 projects in a radial direction of the pin body 331. The projected portions 333 are positioned at opposite sides of the side surface of the pin body 331, so that the pin body 331 is between the projected portions 333.

The projected portions 333 are formed in a rectangular shape, and are formed along the axial direction of the pin body 331.

The heights of the projected portions 333 are each greater than a distance between the inner circumferential surface of the groove portion 1122a (i.e., the inner circumferential surface of the insertion portion 1122 at the first slit portion 1161) and the outer circumferential surface of the pin body 331, as illustrated in FIG. 5. The height of each projected portion 333 indicates a height from the pin body 331 in a radial direction. The outer diameter of the pin body 331 is smaller than the inner diameter of the first slit portions 1161. Hence, the projected portions 333 are formed so that tips of the projected portions 333 are high enough to enter the first slit portions 1161 from the pin body 331. Thus, the projected portions 333 may be inserted into the first slit portions 1161.

The heights of the projected portions 333 are each less than a distance between the inner circumferential surface of the enlarged diameter portion 1122b (i.e., the inner circumferential surface of the insertion portion 1122 at a position of the second slit portion 1162) and the outer circumferential surface of the pin body 331, as illustrated in FIG. 6. Thus, in the enlarged diameter portion 1122b, the projected portions 333 are rotatable in the axial direction together with the pin body 331.

The widths of the projected portions 333 are smaller than or equal to the widths of the first slit portions 1161, as illustrated in FIG. 5. Accordingly, upon the projected portions 333 being inserted into the first slit portions 1161, the projected portions 333 are easily fit into the first slit portions 1161.

As illustrated in FIGS. 3 and 4, it is preferable that the length of each projected portion 333 in the axial direction (X-axis direction) be less than or equal to the length of the first slit portion 1161 in the axial direction (X-axis direction). The lengths of the projected portions 333 in the axial direction (X-axis direction) being less than or equal to the lengths of the first slit portions 1161 in the axial direction (X-axis direction) enables the projected portions 333 to be stably inserted into the first slit portions 1161.

The projected portions 333 are integrally formed on the side surface of the pin body 331. Alternatively, the projected portions 333 may be attached to the side surface of the pin body 331 by welding or the like, as separate members from the pin body 331.

As illustrated in FIGS. 3 and 4, the enlarged portion 334 is disposed on a side surface of an end portion (in the -X-axis direction) of the pin body 331 opposite to the pawl 332 of the pin body 331. The diameter of the enlarged portion 334 in the axial view is larger than the diameter of the pin body 331, as illustrated in FIGS. 3 and 4, and is smaller than the diameter of the enlarged diameter portion 1122b, as illustrated in FIGS. 6 and 7. The outer diameter of the enlarged portion 334 in the axial view is smaller than the sum of the outer diameter of the pin body 331 and the heights of the two projected portions 333.

### [Graduation portion]

As illustrated in FIG. 1, the graduation portion 50 has a base 51 and graduations 52.

The base 51 is fixed to an end of the body 12 opposite to the head 11 (-X-axis direction) and extends from the body 12 toward a direction where the cutout 122a is formed (-Z-axis direction). In the cylindrical axial view of the locking portion 111, the base 51 is formed in a linear fashion. Note that the base 51 may be formed in an arc shape having a curve in a convex fashion in a direction opposite to the head 11. The main shaft 30 is inserted through the base 51.

Graduations 52 are formed on both sides of the base 51 in a cylindrical axial direction of the locking portion 111. The graduations 52 may be formed by printing, engraving, or the like.

The respective members forming the dental torque wrench 1 have been described above. In the following, a tool to be locked to the dental torque wrench 1 will be described. The tool indicates a torque wrench adapter and the like used in the dental torque wrench 1.

FIG. 8 is a front view illustrating an example of a tool 60 that is locked to the dental torque wrench 1. As illustrated in FIG. 8, a tool 60 is a substantially cylindrical member that includes a tool body 61 having a minimum diameter of the tool 60, and a tool head 62 connected to the tool body 61. The tool head 62 has a diameter larger than the diameter of tool body 61.

The tool body 61 has an axially recessed portion 611 in the tool body 61 at a bottom end opposite to the tool head 62. A head of the screw 70 fits into the recessed portion 611, and the screw 70 is connected to the tool 60.

The tool head 62 is formed such that the diameter of the tool head 62 is substantially the same as the inner diameter of the locking portion 111 (see FIG. 1).

The tool head 62 has an annular flange 621 radially projecting from its outer circumferential surface, and an annular projection 622 radially projecting from its outer circumferential surface. The annular projection 622 is disposed close to an end of the tool body 61, and is located at a predetermined interval from the annular flange 621 in a direction opposite to the tool body 61. The tool head 62 has a plurality of grooves 623 extending in an axial direction of the tool head 62 on its outer circumferential surface. The plurality of grooves 623 are formed at substantially equal intervals in a circumferential direction of the tool head 62.

Materials for forming respective members of the dental torque wrench 1 described above are not particularly specified. From the viewpoint of productivity, durability, etc., metals such as titanium, titanium alloy, stainless steel, aluminum, carbon steel, etc. may be used as the materials for forming the main body 10, the main shaft 30, and the graduation portion 50.

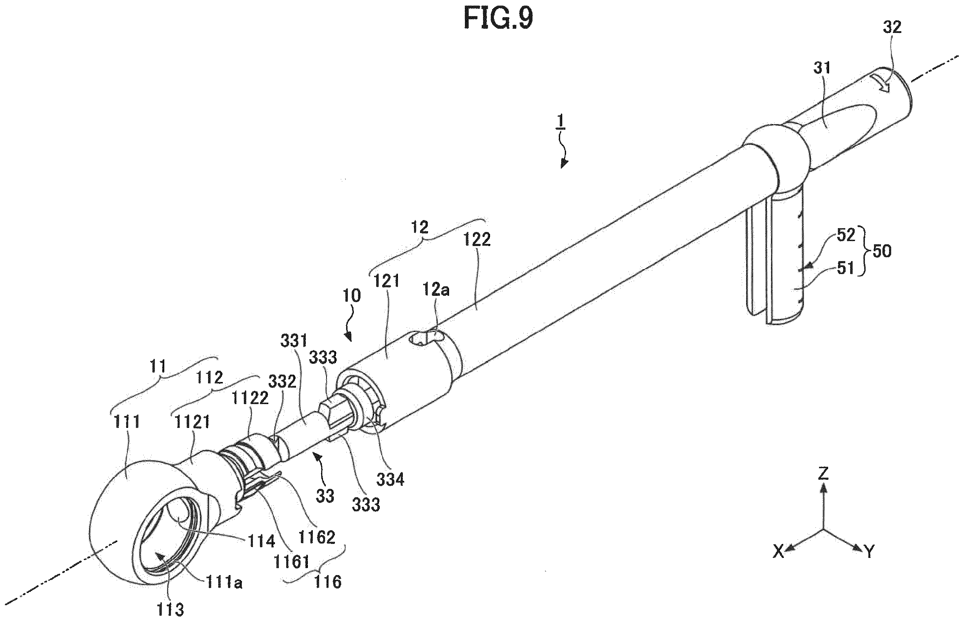

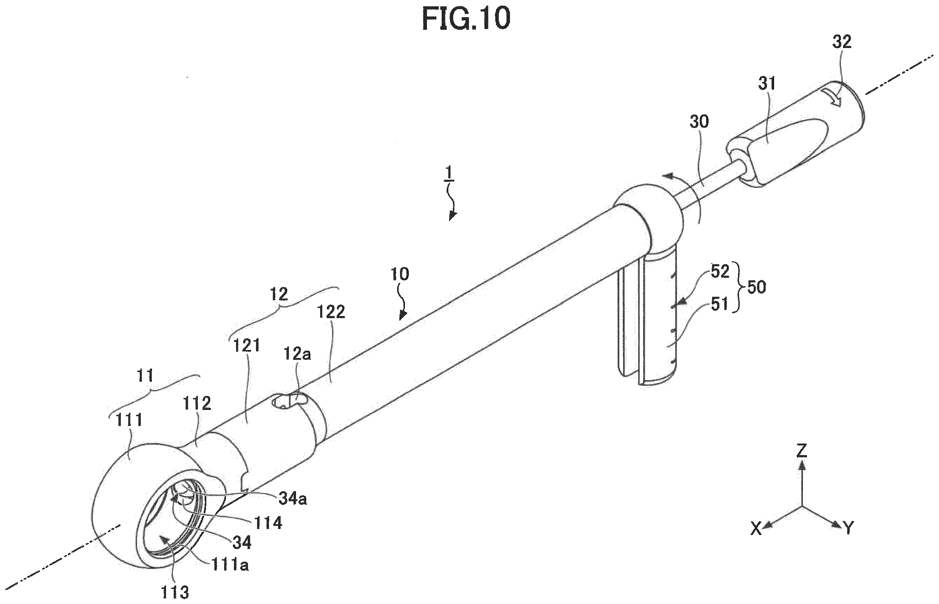

The following illustration indicates a connecting procedure and a disconnecting procedure between the head 11 and the body 12 in the main body 10 of the dental torque wrench 1 with reference to FIGS. 9 and 10.

First, the connecting procedure between the head 11 to the body 12 in the main body 10 of the dental torque wrench 1 will be described. As illustrated in FIG. 9, to connect the head 11 to the body 12, the knob 31 of the main shaft 30 is pushed toward the head 11 to contact the body 12, so that the pin 33 protrudes from the fitting portion 121 of the body 12 toward the head 11. Subsequently, the knob 31 is rotated by substantially 90 degrees around the axis of the main shaft 30 to rotate the projected portions 333 of the pin 33 by substantially 90 degrees around the X-axis together with the main shaft 30. The projected portions 333 of the pin 33 are thus oriented toward the +Z-axis direction. As a result, the projected portions 333 are disposed at positions so as not to be inserted into the first slit portions 1161.

Thereafter, as illustrated in FIG. 10, a connection portion 112 of the head 11 is pushed into the fitting portion 121 of the body 12. The pin 33 is inserted into the insertion portion 1122 while the projected portions 333 and the first slit portions 1161 remain unengaged. Then, a C-ring 13 is disposed to be sandwiched between the groove portion 1122a formed on the outer circumferential surface of the insertion portion 1122 and the groove portion 121a formed on the inner circumferential surface of the fitting portion 121. As a result, the head 11 and the body 12 are connected via the C-ring 13 (see FIG. 2). In this configuration, a preloading force, which preloads the main shaft 30 toward the head 11 (+X-axis direction), is applied from the coil spring 14 to the step 1122c, on the second slit portion 1162 side of the groove portion 1122a of the insertion portion 1122. This preloading force acts on the projected portions 333 of the pin 33. The projected portions 333 thus contact the step 1122c.

Thereafter, when the pin 33 is rotated by substantially 90 degrees around the X-axis together with the main shaft 30 using the knob 31, the projected portions 333 of the pin 33 are inserted into the first slit portions 1161 by a preloading force from the coil spring 14. The head 11 and the body 12 are thus locked by engaging the projected portions 333 with the first slit portions 1161. As illustrated in FIGS. 1 and 2, a tip of the pawl 332 of the main shaft 30 protrudes from an opening 114 of the locking portion 111 into the housing portion 113. In the conditions illustrated in FIGS. 1 and 2, the pawl 332 is orientated toward a direction as illustrated in FIG. 2.

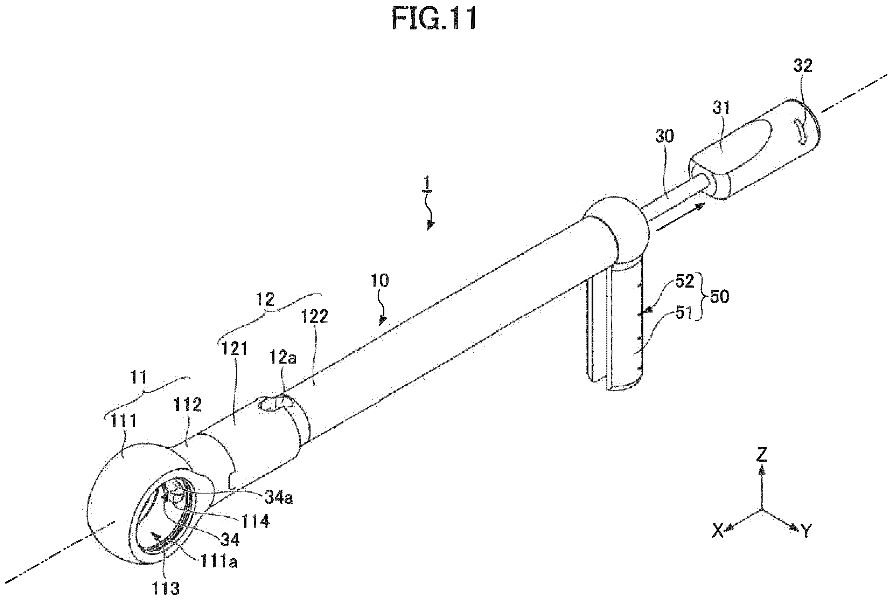

Next, a disconnecting procedure between the head 11 and the body 12 in the dental torque wrench 1 will be described. To disconnect the head 11 from the body 12, the knob 31 of the main shaft 30, with respect to the state in illustrated in FIG. 1, is pulled along the axial direction of the main shaft 30 toward a direction opposite to the head 11 (-X-axis direction), as illustrated in FIG. 11. The projected portions 333 are thus drawn from the first slit portions 1161 together with the pin 33. The tip of the pawl 332 then no longer protrudes into the housing portion 113, and the coil spring 14 contracts due to tensile force of the knob 31. The projected portions 333 of the pin 33 move into the enlarged diameter portion 1122b of the head 11.

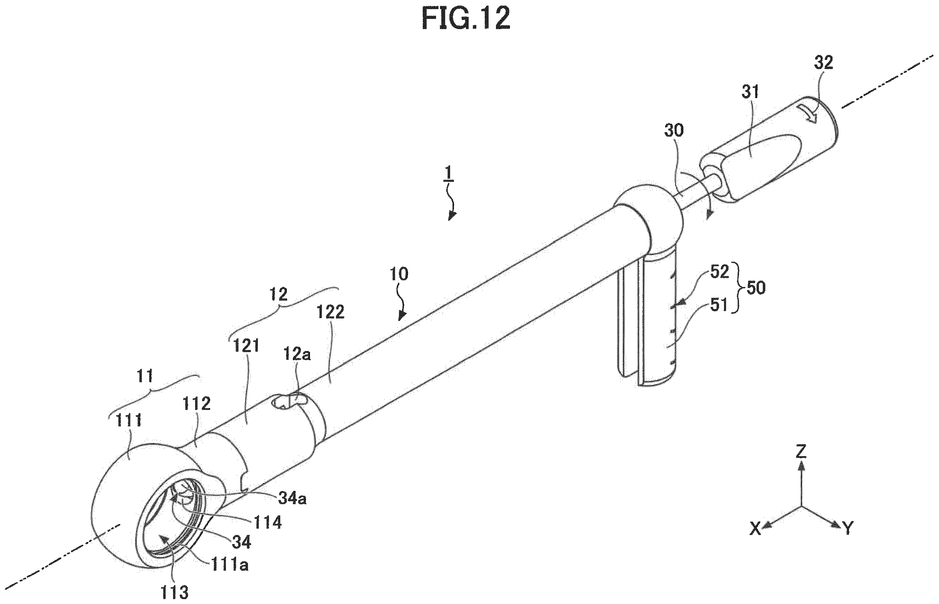

Next, as illustrated in FIG. 12, by operating the knob 31 (see FIG. 2), the pin 33 is rotated, for example, by substantially 90 degrees around the X-axis together with the main shaft 30. As the main shaft 30 rotates, the pin 33 also rotates around the X-axis, for example, by substantially 90 degrees. This changes relative positions of the projected portions 333 of the pin 33 with respect to the enlarged diameter portion 1122b of the head 11. Then, a preloading force, which preloads the projected portions 333 of the pin 33 toward the head 11 (+ X-axis direction) by the coil spring 14, acts on the step 1122c formed on the inner circumferential surface of the insertion portion 1122. As a result, the projected portions 333 contact the step 1122c.

Subsequently, as illustrated in FIG. 9, the head 11 is axially separated from the fitting portion 121 of the body 12 so as to separate the head 11 from the body 12.

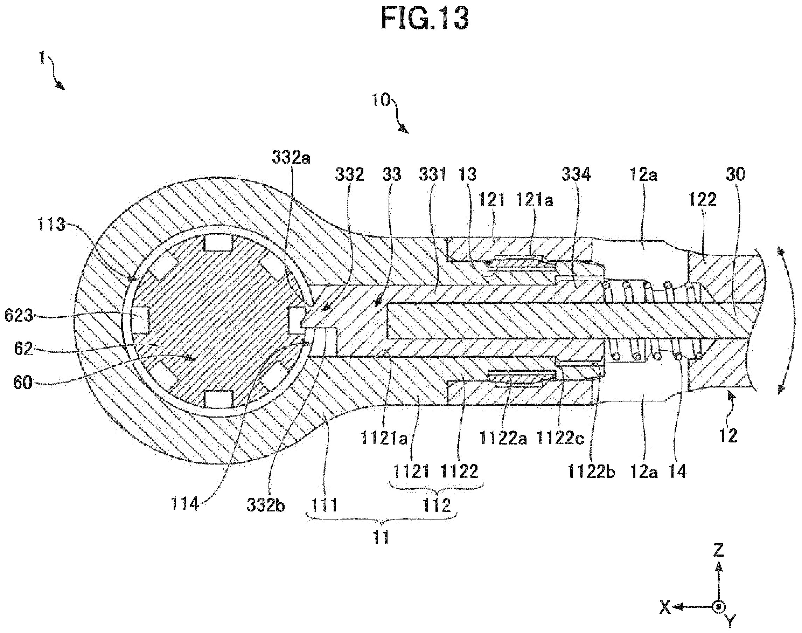

Next, tightening of a screw 70 (see FIG. 8) by the dental torque wrench 1 will be described. FIG. 13 is a partially enlarged sectional view illustrating a state in which a tool 60 is locked to the head 11 of the dental torque wrench 1 illustrated in FIG. 2. Note that FIG. 13 illustrates the vicinity of the head 11. As illustrated in FIG. 13, a fastening tool 60 for a screw 70 (see FIG. 8) is connected to the head 11 of the main body 10 of the dental torque wrench 1. Specifically, the tool head 62 of the tool 60 is inserted into the housing portion 113, which is a space enclosed by a locking portion 111. Then, the annular projection 622 of the tool head 62 (see FIG. 8) is then press-fitted to engage with an annular groove 111a (see FIG. 1) formed on the inner circumferential surface of the locking portion 111. The tool 60 is thus locked to the locking portion 111 to rotate around a cylindrical axis of the locking portion 111.

As described above, the tip of the pawl 332 of the main shaft 30 protrudes from the opening 114 of the locking portion 111 into the housing portion 113 of the locking portion 111. The tip of the pawl 332 engages one of a plurality of grooves 623 formed on the tool head 62, thereby connecting the tool 60 to the head 11.

Next, a force is applied to the knob 31 (see FIG. 1) of the main shaft 30 to bend the main shaft 30 toward a direction where the cutout 122a (see FIG. 2) is formed with the tip of the pawl 332 being pushed into the groove 623. In FIG. 13, a torque in a clockwise direction (illustrated with a broken line in FIG. 2) around the tool 60 is applied to the main shaft 30. As described above, since the main shaft 30 is held in the body 12, torque is transmitted to the main body 10 upon torque being applied to the main shaft 30. Specifically, torque is transmitted to the small diameter portion 122 of the body 12 that supports the main shaft 30. In this case, an engaging surface 332b of the pawl 332 contacts an inner surface of a groove 623 of the tool head 62, and the pawl 332 engages with the groove 623. By connecting the tool 60 to the head 11, the screw 70 (see FIG. 8) coupled to the tool 60 is also coupled to the head 11 via the tool 60. When torque is applied to the screw 70 (see FIG. 8) through the tool 60, which is coupled to the head 11, the screw 70 is tightened by the applied torque (see FIG. 8).

In this state, the main shaft 30, which is exposed to the outside of the body 12 via the cutout 122a, is bent (curved) as illustrated by a broken line in FIG. 2. A graduation 52 of the graduation portion 50 in this state indicates a torque applied to the screw 70 (see FIG. 8), based on the bending degrees of the main shaft 30. Hence, a desired torque may be applied to the screw 70 (see FIG. 8), by applying a torque to the main shaft 30 such that the bent main shaft 30 overlaps a position of a desired torque on the graduation 52. As a result, the screw 70 (see FIG. 8) is tightened with the desired torque.

The pawl 332 has an inclined surface 332a at a tip of the pawl 332. Upon a force being applied to the inclined surface 332a, the force applied to the inclined surface 332a acts toward the axial direction of the main shaft 30. This axial force allows the main shaft 30 to move in the axial direction. When applying a torque to the dental torque wrench 1, an edge of a groove 623 of the tool head 62 contacts the inclined surface 332a to apply force to the inclined surface 332a. That is, in FIG. 13, when a torque in a counterclockwise direction around the tool 60 is applied to the dental torque wrench 1, the main shaft 30 moves in an opposite axial direction from the locking portion 111 (in -X-axis direction) along the outer circumferential surface of the tool head 62 and the inner surface of the groove 623. The pawl 332 is then drawn from the groove 623 to disengage the pawl 332 from the groove 623. As a result, torque is no longer applied to the screw 70 (see FIG. 8).

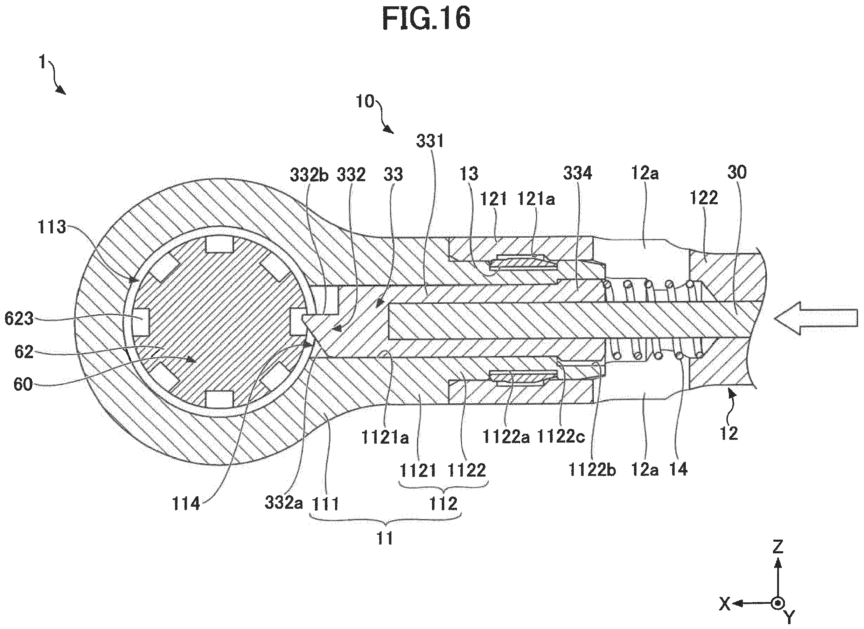

Thus, the application of a torque to the dental torque wrench 1 may be adjusted so as not to apply torque to the screw 70 (see FIG. 8). Upon a torque being applied in a direction toward the engaging surface 332b of the pawl 332 (clockwise direction around the tool 60 in FIG. 13), the pawl 332 engages with the tool 60 to rotate the tool 60, thereby applying a torque to the screw 70 through the tool 60. However, upon a torque being applied in a direction toward the inclined surface 332a of the pawl 332 (clockwise direction around the tool 60 in FIG. 16), the pawl 332 disengages from tool 60, thereby causing the dental torque wrench 1 to spin out. As a result, no torque is applied to the screw 70. As described above, the dental torque wrench 1 has a so-called ratchet mechanism (feed function) operable by the pawl 332.

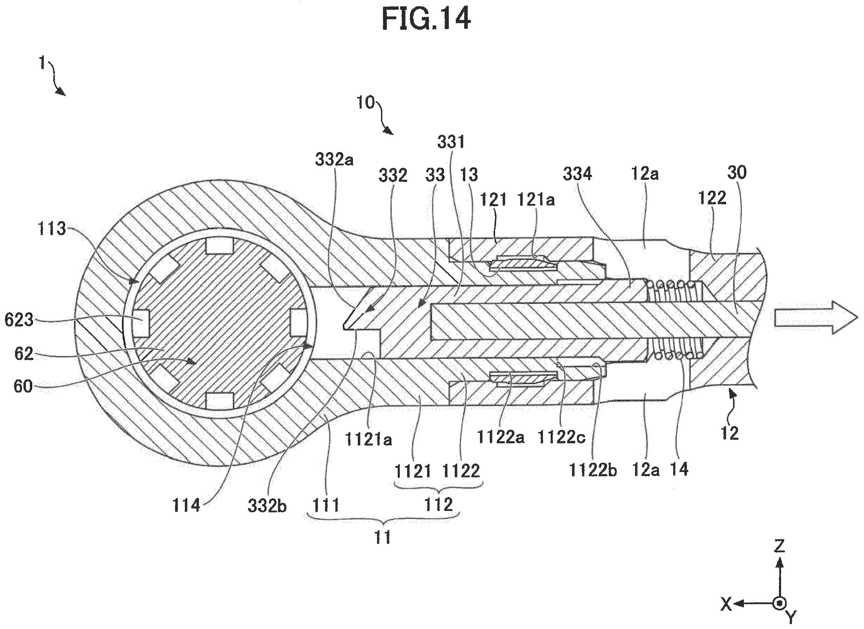

Next, a reversing procedure of the ratchet mechanism of the dental torque wrench 1 will be described with reference to FIGS. 14 to 16. In the following description, the pawl 332 in an initial state is orientated toward a direction as illustrated in FIG. 2. In this initial state, the dental torque wrench 1 is configured to rotate the tool 60 in a clockwise direction as illustrated in FIGS. 14 to 16.

First, as illustrated in FIG. 14, by holding the knob 31 (see FIG. 2) of the main shaft 30, the main shaft 30 is pulled in the axial direction of the main shaft 30 toward a position opposite to the head 11 (in the -X-axis direction). The tip of the pawl 332 thus no longer protrudes into the housing portion 113. In this state, the coil spring 14 contracts by tensile force of the knob 31, and the projected portions 333 of the pin 33 are moved into the enlarged diameter portion 1122b of the head 11.

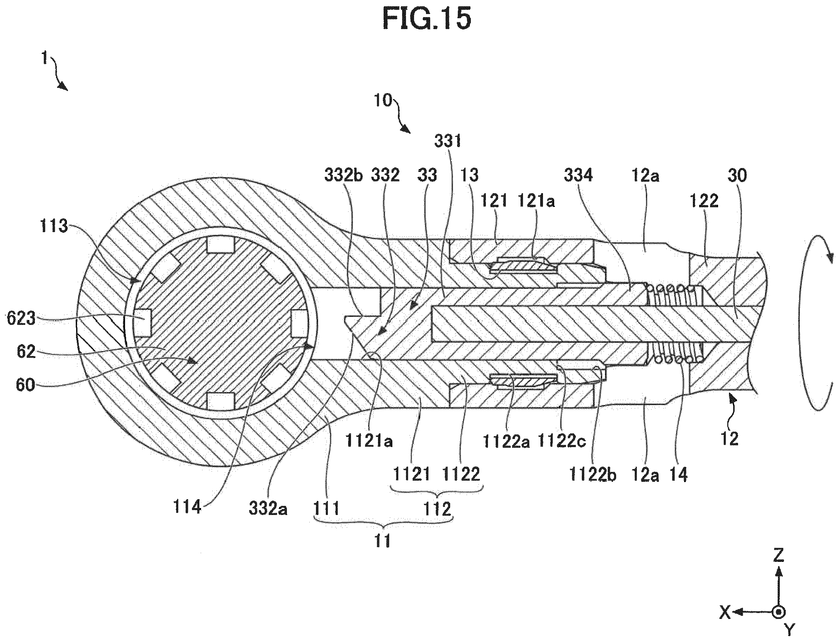

Next, as illustrated in FIG. 15, the knob 31 (see FIG. 2) is rotated, for example, by substantially 180° around the X-axis of the main shaft 30. As the main shaft 30 rotates, the pin 33 also rotates, for example, by substantially 180° around the X-axis, so that relative positions of the projected portions 333 of the pin 33 with respect to the enlarged diameter portion 1122b of the head 11 are rotated by 180°.

Upon pulling of the knob 31 (see FIG. 2) being stopped to release the tension of the main shaft 30, a preloading force that preloads the main shaft 30 toward the head 11 (+X-axis direction) is applied by a coil spring 14. The projected portions 333 of the pin 33 thus fit into the first slit portions 1161 of the head 11. The tip of the pawl 332 of the main shaft 30 is pushed out from the opening 114 to the housing portion 113 in a direction rotated by, for example, approximately 180 degrees from the state in FIG. 13, and the protruded tip of the pawl 332 is pushed against the outer circumferential surface of the tool head 62.

In this state, the dental torque wrench 1 is rotated with respect to the tool 60 to locate the tip of the pawl 332 at a groove 623 of the tool head 62. The tip of the pawl 332 located at a groove 623 is pushed into the groove 623 by a preloading force from the coil spring 14. In this state, the dental torque wrench 1 is switched to rotate the tool 60 in a counterclockwise direction in the figure to reverse the ratchet mechanism.

In this manner, the dental torque wrench 1 includes projected portions 333 on the pin body 331 of the pin 33, the first slit portions 1161 are formed on the insertion portion 1122 of the connection portion 112 of the head 11, and a C-ring 13 is provided in the groove portion 1122a of the insertion portion 1122 of the head 11. Upon the projected portions 333 being inserted into the first slit portions 1161, the head 11 and the body 12 that are attached are locked by the C-ring 13 disposed in the groove portion 1122a to press the inner circumferential surface of the fitting portion 121. Accordingly, even for a force being applied to the body 12 in the direction of separating the head 11 from the body 12 that would deform the C-ring 13, one of the projected portions 333 presses against the inner circumferential surface of the C-ring 13, and the projected portion 333 does not allow the opening of the C-ring 13 to close. Thus, the C-ring 13 does not deform. The insertion portion 1122 of the connection portion 112 may thus be prevented from coming off from the fitting portion 121 of the body 12. Accordingly, upon attaching the head 11 and the body 12, the head 11 and the body 12 may be prevented from being detached, and attachment of the head 11 and the body 12 may be stably maintained.

Further, upon detaching the head 11 and the body 12, the projected portions 333 may be drawn from the first slit portions 1161 by pulling the main shaft 30 and rotating the pin 33. Upon a force being applied to the body 12 in the direction of detaching the body 12 from the head 11, the opening of the C-ring 13 that is about to deform is allowed to close, and the C-ring 13 is allowed to deform. Thus, the insertion portion 1122 of the connection portion 112 may be easily drawn from the fitting portion 121 of the body 12. Hence, when it is not necessary to maintain attachment of the head 11 and the body 12, the head 11 and the body 12 may be readily detached.

Thus, the dental torque wrench 1 may stably maintain attachment of the head 11 and the body 12 upon the head 11 and the body 12 being attached, and the dental torque wrench 1 may readily detach the head 11 and the body 12 upon the head 11 and body 12 being detached. Since the dental torque wrench 1 has good operability, working efficiency in implant treatment may be improved.

The dental torque wrench 1 may be readily processed by simply providing the first slit portions 1161 on the head 11 and simply providing the projected portions 333 on the pin 33. Since the processing of the dental torque wrench 1 is simple, manufacturing costs may be reduced. Further, because the head 11 may be separated from the body 12, the projected portions 333, the first slit portions 1161, and the like may be easily cleaned, thereby improving cleanability.

In the dental torque wrench 1, a groove portion 1122a configured to receive a C-ring 13 is formed in a circumferential direction on the side surface of the insertion portion 1122, and the groove portion 1122a is provided with first slit portions 1161. In this configuration, the C-ring 13 may be stably held by the groove portion 1122a. Thus, even when the pin 33 is inserted or removed repeatedly, the position of the C-ring 13 may be prevented from fluctuating. Further, upon the projected portions 333 being inserted into the first slit portions 1161, deforming of the C-ring 13 may more reliably be prevented by the C-ring 13 being held by the groove portion 1122a.

The dental torque wrench 1 has one end portion located close to the locking portion 111 and the other end portion located opposite to the locking portion 111. The dental torque wrench 1 includes an enlarged diameter portion 1122b at the other end portion into which the pin 33 is inserted, and also includes a step 1122c between the inner circumferential surface of the groove portion 1122a and the inner circumferential surface of the enlarged diameter portion 1122b. Upon the pin 33 being pulled, tips of the projected portions 333 may be hooked on the step 1122c, thereby allowing the pin 33 to remain in a pulled state. Since the heights of the projected portions 333 from the pin body 331 in the radial direction are smaller than the distance between the inner circumferential surface of the enlarged diameter portion 1122b and the pin body 331, the projected portion 333 may be rotated axially together with the pin 33 by the enlarged diameter portion 1122b. This reduces pulling force of the pin 33 and also reduces load on the coil spring 14. Further, the enlarged diameter portion 1122b is disposed at the other end portion of the insertion portion 1122. Thus, when the projected portions 333 are not inserted into the first slit portions 1161, the projected portions 333 of the pin 33 may be simply pulled out to a position of the enlarged diameter portion 1122b, thereby facilitating operability.

The dental torque wrench 1 has second slit portions 1162 on the side surface of the enlarged diameter portion 1122b of the insertion portion 1122. Since the second slit portions 1162 are connected to the first slit portions 1161, the projected portions 333 may be readily inserted into and drawn from the first slit portions 1161 through the second slit portions 1162.

The dental torque wrench 1 includes the pin body 331 having one end portion located close to the head 11 and the other end portion located opposite to the head 11. The dental torque wrench 1 includes an enlarged portion 334 at the other end portion of the pin body 331. The outer diameter of the enlarged portion 334 is larger than the outer diameter of the pin body 331 and smaller than the inner diameter of the enlarged diameter portion 1122b in the radial direction. The pin 33 being provided with an enlarged portion 334 reduces a gap between the other end portion of the pin body 331 and the insertion portion 1122 upon the projected portions 333 being inserted into the first slit portions 1161. As a result, the connection between the insertion portion 1122 and the pin 33 may be stabilized.

The dental torque wrench 1 has a pawl 332 at the tip of the pin body 331. Since the pawl 332 is exposed to the head 11 to transmit a rotational force in one direction to the tool 60, the tool 60 may only be rotated in one direction, thereby preventing reverse rotation. Further, the dental torque wrench 1 may readily and reliably switch the orientation of the pawl 332 by simply drawing the pin 33 from the first slit portions 1161 and rotating the pin 33, for example, by approximately 180 degrees. Thus, it is possible to readily and reliably reverse the ratchet mechanism.

The following illustrates modification of the embodiment. According to the modification of the embodiment, the tips of the projected portions 333 may be formed in an arc or may have rounded corners when viewed from a longitudinal (Y-axis) direction of the pin 33. The tips of the projected portions 333 may be formed in a trapezoid or in a triangle in the axial view of the pin 33.

According to the modification of the embodiment, the number of projected portions 333 may be one or more than three.

According to the embodiment, the body 12 is illustrated having a cylindrical member; however, the body 12 may be non-cylindrical insofar as the body 12 includes the main shaft 30. For example, the body 12 may be a tubular body having a polygonal cross section when viewed in the axial direction. Further, the body 12 is not necessarily a complete tubular shape, and may have a gap formed along the axial (X-axis) direction.

According to the embodiment, the outer diameter of the fitting portion 121 of the body 12 is larger than the outer diameter of the other portions of the body 12; however, the outer diameter of the fitting portion 121 of the body 12 may be substantially the same as the outer diameters of the other portions.

According to the embodiment, the main shaft 30 is made of a cylindrical member; however, the main shaft 30 may have a shape other than a cylindrical shape having a cross-section such as a polygonal shape.

According to the modification of the embodiment, the second slit portions 1162 formed from the groove portion 1122a to the other end portion located opposite to the locking portion 111 may not necessarily be formed.

According to the embodiment, a C-ring 13 is used as a clamp member to secure the connection portion 112 of the head 11 to the fitting portion 121 of the body 12; however, the clamp member may simply be used to fit the insertion portion 1122 into the fitting portion 121. For example, other than the C-ring 13, the clamp member may be a polygon member having a predetermined opening in the axial view of the pin 33.

Although the present embodiment has been described as above, the above-described embodiment is merely presented as examples and the present embodiment is not limited to the above-described examples. Various other modes and combinations, omissions, substitutions, alterations, and the like may be applied to the above-described embodiments without departing from the gist of the invention. These embodiments and modifications thereof are included in the scope and gist of the invention, and are included in the invention described in the claims and the equivalent scope thereof.

## Claims

1. A dental torque wrench (1) comprising:
a main body (10) having a head (11), and a body (12), the head (11) being coupled to a tool (60), and the body (12) being detachably coupled to the head (11) ;
a main shaft (30) configured to be held in a state of being inserted into the body (12);
a pin (33) having a pin body (331), the pin (33) being disposed at one end portion of the main shaft (30), the one end portion of the main shaft (30) being located close to the head (11), and the pin (33) extending along an axial direction of the main shaft (30) ;
a clamp member (13) having a predetermined opening in an axial view of the pin (33), the clamp member (13) being disposed to be sandwiched between an inner circumferential surface of the body (12) and an outer circumferential surface of an insertion portion (1122) of the head (11), the insertion portion (1122) being inserted into an internal space of the body (12);
at least one projected portion (333) disposed on a side surface of the pin body (331), the projected portion (333) being projected in a radial direction of the pin body (331); and
a first slit portion (1161) formed on a side surface of the insertion portion (1122), the first slit portion (1161) being configured to allow the projected portion (333) to be detachably inserted in the first slit portion (1161), wherein
upon coupling of the head (11) and the body (12), the pin (33) is inserted into the insertion portion (1122) of the head (11) without allowing the projected portion (333) to engage with the first slit portion (1161), the clamp member (13) is disposed to be sandwiched between the inner circumferential surface of the body (12) and the outer circumferential surface of the insertion portion (1122), and the projected portion (333) subsequently engages with the first slit portion (1161) to lock the head (11) and the body (12).

2. The dental torque wrench (1) according to claim 1, wherein
the insertion portion (1122) has a groove portion (1122a) on a side surface of the insertion portion (1122) along a circumferential direction, the groove portion (1122a) being configured to receive the clamp member (13), and the first slit portion (1161) is formed in the groove portion (1122a).

3. The dental torque wrench (1) according to claim 2, wherein
the insertion portion (1122) has one end portion located close to the head (11) and another end portion located opposite to the head (11), and the insertion portion (1122) includes an enlarged diameter portion (1122b) at the other end portion into which the pin (33) is inserted, the enlarged diameter portion (1122b) having an inner diameter larger than an inner diameter of the groove portion (1122a), and wherein
the insertion portion (1122) includes a step (1122c) between an inner circumferential surface of the groove portion (1122a) and an inner circumferential surface of the enlarged diameter portion (1122b).

4. The dental torque wrench (1) according to claim 3, further comprising:
a second slit portion (1162) formed on a side surface of the enlarged diameter portion (1122b), the second slit portion (1162) being connected to the first slit portion (1161).

5. The dental torque wrench (1) according to claim 3 or 4, wherein
the pin body (331) has one end portion located close to the head (11) and another end portion located opposite to the head (11), and the pin (33) includes an enlarged portion (334) at the other end portion of the pin body (331), wherein
the enlarged portion (334) has a diameter greater than a diameter of the pin body (331) and smaller than a diameter of the enlarged diameter portion (1122b).

6. The dental torque wrench (1) according to any one of claims 1 to 5, wherein
the pin (33) includes a pawl (332) disposed at a tip of the pin body (331), and the pawl (332) is exposed into the head (11) to propagate a rotational force to the tool (60) in one direction.
